# EUROPEAN PATENT APPLICATION

(11) **EP 4 831 624 A1**
(43) Date of publication of application: **28.10.2026**
(21) Application number: 24906156.5
(22) Date of filing: 09.12.2024
(51) Int. Cl.: G01N 23/00, A61B 6/00

(54) **RADIATION IMAGING DEVICE, CT IMAGING DEVICE, AND METHOD THEREOF**

(30) Priority: 21.12.2023 CN 202311774400
(71) Applicant: Tsinghua University, Beijing 100084 (CN); Nuctech Company Limited, TongFang Building Shuangqinglu Haidian District Beijing 100084 (CN)
(72) Inventor: CHEN, Zhiqiang, Beijing 100084 (CN); ZHANG, Li, Beijing 100084 (CN); LI, Xinbin, Beijing 100084 (CN); WANG, Zhentian, Beijing 100084 (CN); HUANG, Qingping, Beijing 100084 (CN); SUN, Yunda, Beijing 100084 (CN); SHEN, Le, Beijing 100084 (CN); FENG, Bo, Beijing 100084 (CN)
(74) Representative: Gunzelmann, Rainer
(86) International application number: PCT/CN2024/137723
(87) International publication number: WO 2025/130672

(57) **Abstract**

A radiation imaging apparatus, a CT imaging apparatus, a radiation imaging method, and a CT imaging method. The radiation imaging apparatus includes: a radiation source (S), a detector (DT), and one or more gratings (G0, G1, G2) arranged in an inspection channel defined by the radiation source (S) and the detector (DT). The gratings (G0, G1, G2) are configured to block only a portion of the radiation beam such that the portion of the radiation beam passes through the gratings (G0, G1, G2) before being received by the detector (DT).

## Description

This application claims the benefit of the priority to Chinese Patent Application No. 202311774400.3 filed on December 21, 2023, the entire contents of which are incorporated herein by reference.

### TECHNICAL FIELD

The present disclosure relates to a field of detection technology, and more specifically, relates to a radiation imaging apparatus and method, and a CT imaging apparatus and method.

### BACKGROUND

Related X-ray Computed Tomography (CT) imaging apparatuses are usually implemented based on existing X-ray absorption imaging. Their core components include the X-ray source and the detector, which may obtain absorption images reflecting attenuation characteristics of X-rays passing through substances. Such absorption images exhibit strong discriminating capability for high-density substances having strong X-ray absorption capability, and provide excellent imaging effects for substances such as metals, bones, and the like. Therefore, absorption images have been widely used in security inspection fields for identifying contraband and in medical fields for diagnosing bone diseases. However, for substances composed of light elements (such as carbon, hydrogen, oxygen, nitrogen, etc.), the signals in absorption images are weak, resulting in significant limitations. For example, in the field of security inspection, it is difficult to observe contraband such as drugs shielded by metal, and in the medical field, it is difficult to observe pathological tissues in the lungs. In summary, in order to improve the effectiveness of security inspection and medical diagnosis, new technologies and new products that help enhance the image discriminating capability for weakly absorbing substances may be explored.

### SUMMARY

According to an aspect of the present disclosure, a radiation imaging apparatus is provided, including: a radiation source configured to emit a radiation beam; a detector configured to receive the radiation beam from the radiation source, where the radiation source and the detector define an inspection channel; and one or more gratings arranged in the inspection channel and between the radiation source and the detector; where the one or more gratings are configured such that a portion of the radiation beam passes through the one or more gratings before being received by a first portion of the detector, and another portion of the radiation beam is received by a second portion of the detector without passing through the one or more gratings.

In an embodiment, the first portion of the detector includes a high-energy detector, and the second portion of the detector includes a low-energy detector.

In an embodiment, the radiation imaging apparatus is configured to construct at least a low-energy absorption image based on a radiation beam signal acquired by the low-energy detector, construct a high-energy absorption image based on a radiation beam signal acquired by the high-energy detector, and combine the high-energy absorption image and the low-energy absorption image to construct an information-enhanced comprehensive image.

In an embodiment, the one or more gratings include a periodic structure, and the periodic structure is configured to diffract the radiation beam to generate an interference fringe.

In an embodiment, the first portion of the detector and the second portion of the detector are integrated, or the first portion of the detector and the second portion of the detector are separated from each other.

In an embodiment, the one or more gratings include: a first grating arranged adjacent to and parallel to the detector and spaced apart from the detector by a first distance, where the radiation beam irradiates an object under inspection after passing through the first grating; and/or a second grating arranged adjacent to and parallel to the detector and spaced apart from the detector by a second distance different from the first distance, where the radiation beam irradiates the object under inspection after passing through the second grating; or a second grating arranged adjacent to and parallel to the detector and spaced apart from the detector by a third distance, where the radiation beam passes through the second grating after passing through the object under inspection; and/or a third grating arranged adjacent to and parallel to the detector and spaced apart from the detector by a fourth distance different from the third distance, where the radiation beam passes through the third grating after passing through the object under inspection.

In an embodiment, the radiation imaging apparatus includes a first grating, a second grating, and a third grating, where the first grating, the second grating, and the third grating are parallel to each other.

In an embodiment, the periodic structure of the grating is a channel-type structure or a slit-type structure.

In an embodiment, the radiation imaging apparatus is configured to generate, based on signals detected by the detector, at least one of an absorption image, a phase image, or a small-angle scattering image related to at least a partial region of an object under inspection in the inspection channel.

In an embodiment, the one or more gratings are configured as a planar grating or an arcuate surface grating.

According to an aspect of the present disclosure, a CT imaging apparatus is provided, including: a rotating ring configured to be rotatable; and the radiation imaging apparatus described above, where the radiation imaging apparatus is arranged on the rotating ring and configured to rotate with the rotating ring.

According to an aspect of the present disclosure, a radiation imaging method is provided, including: imaging an object under inspection using the radiation imaging apparatus described above, where the object under inspection moves along the inspection channel to sequentially pass through the first portion and the second portion of the detector, and at least one of an absorption image, a phase image, or a small-angle scattering image related to at least a partial region of the object under inspection is generated based on signals detected by the first portion and the second portion of the detector.

According to an aspect of the present disclosure, a CT imaging method is provided, including: imaging an object under inspection using the CT imaging apparatus described above, where the object under inspection moves along the inspection channel to sequentially pass through the first portion and the second portion of the detector, and at least one of a three-dimensional absorption image, a three-dimensional phase image, or a three-dimensional small-angle scattering image related to at least a partial region of the object under inspection is generated based on signals detected by the first portion and the second portion of the detector.

It should be understood that the content described in this section is not intended to identify key or essential features of embodiments of the present disclosure, nor is it intended to limit the scope of the present disclosure. Other features of the present disclosure will become readily understandable through the following description.

### BRIEF DESCRIPTION OF THE DRAWINGS

The accompanying drawings are provided for better understanding of the present solution and do not constitute any limitations to the present disclosure, in which:
FIG. 1 shows examples of an arrangement of gratings in a radiation imaging apparatus;
FIG. 2 shows a radiation imaging apparatus according to an embodiment of the present disclosure;
FIG. 3 shows a radiation imaging apparatus according to another embodiment of the present disclosure;
FIG. 4 shows a radiation imaging apparatus according to another embodiment of the present disclosure, where a detector includes two separate portions;
FIG. 5 shows two-dimensional images for absorption *T* (a), phase Φ (b), and small-angle scattering *D* (c), as well as corresponding XY-plane slice images of three-dimensional images for linear attenuation coefficient *µ* (d), refractive index decrement *δ* (e), and linear diffusion coefficient *ζ* (f) reconstructed using an FBP algorithm from 360 projections acquired over one rotation (corresponding to Equation (3) of the technical disclosure document); and
FIG. 6 shows stained histological microscopic images (a1 to a6) of human breast specimens 1 to 6, as well as corresponding XZ-plane slice images of reconstructed three-dimensional images for linear attenuation coefficient *µ* (b1 to b6) and XZ-plane slice images of reconstructed three-dimensional images for refractive index decrement *δ* (c1 to c6).

### DETAILED DESCRIPTION OF EMBODIMENTS

To more clearly describe the objectives, technical solutions, and advantages of the present disclosure, embodiments of the present disclosure will be described in detail below with reference to the accompanying drawings. It should be understood that the following description of embodiments is intended to explain and illustrate the general concept of the present disclosure and should not be construed as limiting the present disclosure. In the specification and the accompanying drawings, the same or similar reference numerals denote the same or similar components or members. For purposes of clarity, the drawings are not necessarily drawn to scale, and certain well-known components and structures may be omitted from the drawings.

Unless otherwise defined, technical or scientific terms used herein shall have the meanings commonly understood by a person having ordinary skill in the art to which the present disclosure pertains. Terms such as "first", "second", and similar expressions used herein do not indicate any order, quantity, or importance, but are merely used to distinguish different components. Terms "a" and "an" do not exclude the presence of multiple. Terms such as "comprising", "including", or similar expressions indicate that the element or item preceding the word encompasses the element or item listed thereafter as well as equivalents thereof, but does not exclude additional elements or items. Terms such as "connected", "coupled", or similar expressions are not limited to physical or mechanical connections and may include electrical connections, whether direct or indirect. Terms such as "upper", "lower", "left", "right", "top", "bottom", and the like are used only to indicate relative positional relationships, and such relative positional relationships may change when the absolute position of the described object changes. When an element such as a layer, film, region, or substrate is described as being arranged "on" or "under" another element, the element may be directly on or under the other element, or one or more intermediate elements may be present therebetween.

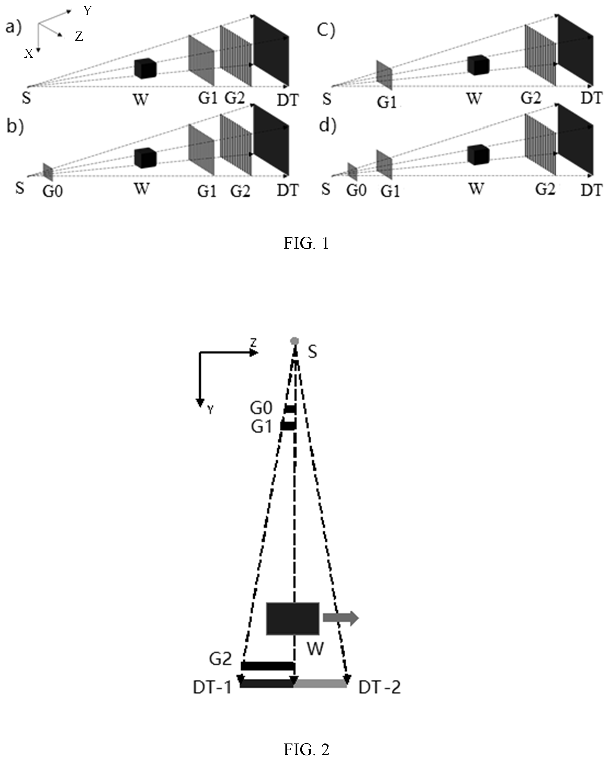

In (a), (b), (c), and (d) of FIG. 1, various grating imaging arrangements are shown, and G1, G2, and G3 represent three gratings, which are respectively arranged between a source and an object under inspection or between the object under inspection and a detector as shown in (a), (b), (c), and (d) of FIG. 1.

In grating imaging technology, both the wave nature and the particle nature of X-rays may be utilized to acquire multi-characteristic information including three types of information, namely absorption information, phase information, and small-angle scattering information, where the absorption information and the phase information respectively correspond to an imaginary part *β* and a real part *δ* of a complex refractive index n. $n = 1 - δ + iβ$ (1) where a linear attenuation coefficient µ has a linear relationship with the imaginary part β (λ represents a wavelength of the X-rays). $μ = \frac{4 πβ}{λ}$ (2)

The three types of characteristic information of absorption *T*, phase Φ, and small-angle scattering *D* may be respectively regarded as integrals of the linear attenuation coefficient µ, the refractive index decrement *δ*, and the linear diffusion coefficient ζ (where *L* represents a propagation path of X-rays, and *c*_{Φ} and *c_{D}* are both constant values related to the imaging system). $\left\{\begin{matrix}T = exp \left(-{\int }_{L} μ \left(\vec{r}\right) d \vec{r}\right) \\ Φ = {c}_{Φ} ⋅ \frac{\partial }{\partial x} \left({\int }_{L} δ \left(\vec{r}\right) d \vec{r}\right) \\ D = {c}_{D} ⋅ exp \left(-{\int }_{L} ζ \left(\vec{r}\right) d \vec{r}\right)\end{matrix}\right.$ (3)

Considering that X-rays generally exhibit an energy spectrum distribution, and assuming that a normalized energy spectrum distribution is represented by S(E), Equation (3) may be expressed as: $\left\{\begin{matrix}T = {\int }_{{E}_{0}}^{{E}_{1}} S \left(E\right) ⋅ exp \left(-{\int }_{L} μ \left(\vec{r}, E\right) d \vec{r}\right) dE \\ Φ = {c}_{Φ} ⋅ {\int }_{{E}_{0}}^{{E}_{1}} S \left(E\right) ⋅ \frac{\partial }{\partial x} \left({\int }_{L} δ \left(\vec{r}, E\right) d \vec{r}\right) dE \\ D = {c}_{D} ⋅ {\int }_{{E}_{0}}^{{E}_{1}} S \left(E\right) ⋅ exp \left(-{\int }_{L} ζ \left(\vec{r}, E\right) d \vec{r}\right) dE\end{matrix}\right.$ (4)

In (a) of FIG. 1, the grating G1 may function to form a self-imaging fringe pattern in a downstream optical path. When X-rays interact with a substance, the self-imaging fringe pattern may undergo local distortion and deformation. Such deformation occurs on a micrometer scale or even a sub-micrometer scale, which is beyond the resolution that may be achieved by ordinary detectors. Therefore, grating G2 amplifies the signal. By observing variations in the intensity of the detector signal, three characteristic images of absorption, phase, and small-angle scattering may be extracted. When a focal spot of the X-ray source is sufficiently small (e.g., in a synchrotron radiation source or a micro-focus X-ray source), only gratings G1 and G2 are required, as shown in (a) and (c) of FIG. 1

When the focal spot of the X-ray source has a large size (e.g., in an existing X-ray source), the resulting blurring effect may deteriorate the imaging effect. Therefore, a grating G0 may be added downstream of the X-ray source. Due to the presence of grating G0, the radiation beams emitted by the X-ray source may be converted into a series of partially coherent micrometer-scale linear radiation beams, as shown in (b) and (d) of FIG. 1.

An object under inspection W may be positioned between grating G0 and grating G1 (as shown in (b) of FIG. 1), or between grating G1 and grating G2 (as shown in (c) and (d) of FIG. 1).

When the object under inspection W is positioned between gratings G0 and G1, as shown in (b) of FIG. 1, in this configuration, stronger phase signals and small-angle scattering signals may be obtained, resulting in higher imaging contrast and sensitivity. However, this configuration also has certain disadvantages, that is, grating G1 has a large area and incurs higher cost. In addition, X-rays transmitted through the object under inspection W may be blocked by gratings G1 and G2 before being received by the detector, resulting in low X-ray utilization efficiency.

When the object under inspection W is positioned between gratings G1 and G2, as shown in (c) and (d) of FIG. 1, in this configuration, grating G1 is located close to the light source and may have a small area and low cost. Furthermore, X-rays transmitted through the object under inspection W are blocked only by grating G2, resulting in higher X-ray utilization efficiency. However, the phase signals and small-angle scattering signals obtained in this configuration are somewhat weaker. Nevertheless, for certain strongly scattering substances, this is less likely to cause detector signal saturation.

The absorption image (the above T) reflects attenuation characteristics of the substance with respect to X-rays, corresponding to the imaginary part *β* of the complex refractive index of the substance for X-rays (complex refractive index *n* = 1 - *δ* + *iβ*). The phase image reflects refraction effects of the substance on X-rays, corresponding to the real part *δ* of the complex refractive index of the substance for X-rays. For weakly absorbing substances, the phase image advantageously provides higher contrast than the absorption image, and *δ* may be higher than *β* by 2 to 3 orders of magnitude. Accordingly, weakly absorbing substances may be displayed more clearly through the phase image. The small-angle scattering image reflects a mesoscopic structural information of the substance and is highly sensitive to differences at the micrometer scale or sub-micrometer scale, such as porosity and multi-fiber structures. Accordingly, the small-angle scattering image may break through the resolution limitations of absorption imaging. The phase image and the small-angle scattering image are valuable supplements to the absorption image, which may effectively improve the overall imaging effect and resolving capability for the object under inspection W, and enhance the adaptability and accuracy of the inspection process for different substances.

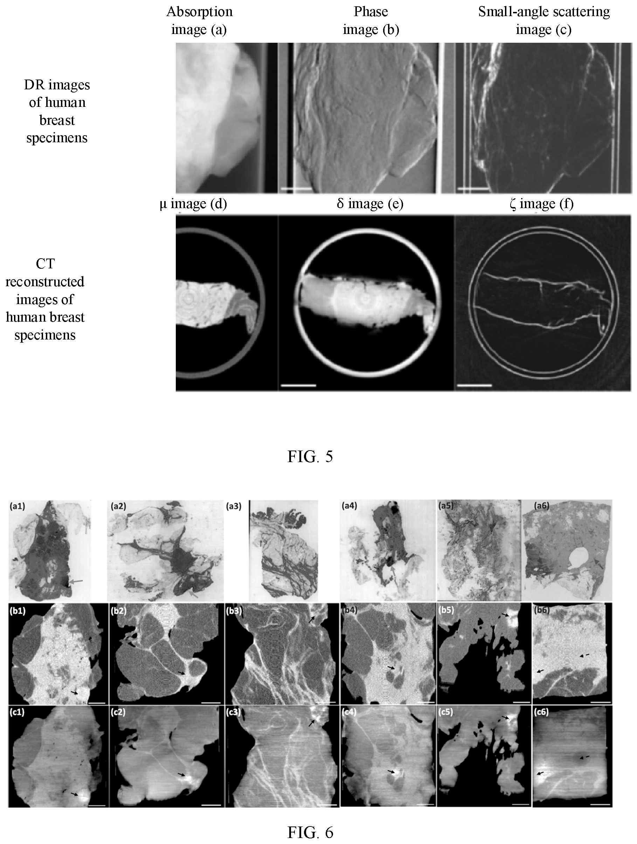

FIG. 5 shows two-dimensional images for absorption *T* (a), phase Φ (b), and small-angle scattering *D* (c), as well as corresponding XY-plane slice images of three-dimensional images for linear attenuation coefficient *µ* (d), refractive index decrement *δ* (e), and linear diffusion coefficient *ζ* (f) reconstructed using an FBP algorithm from 360 projections acquired over one rotation (corresponding to Equation (3) of the technical disclosure document). The absorption image (corresponding to the linear attenuation coefficient *µ* ) reflects attenuation characteristics of the substance. The phase image (corresponding to the refractive index decrement *δ* in the complex refractive index) provides higher contrast for weakly absorbing substances and reflects a texture information of internal and external structures of the object under inspection W. The small-angle scattering image (corresponding to the linear diffusion coefficient *ζ* ) reflects the mesoscopic structural information of the substance and reflects microscopic structures such as porosity and multi-fiber structures at the micrometer/sub-micrometer scale.

FIG. 6 shows stained histological microscopic images (a1 to a6) of human breast specimens 1 to 6, as well as corresponding XZ-plane slice images of reconstructed three-dimensional images for linear attenuation coefficient *µ* (b1 to b6) and XZ-plane slice images of reconstructed three-dimensional images for refractive index decrement *δ* (c1 to c6).

In specimens 1, 2, 4, and 5, the arrows indicate tumor lesions (darkened regions); in specimen 3, the arrow indicates pectoral muscle tissue; in specimen 6, the arrow indicates a necrotic region (darkened region).

In all specimen images, fat tissues may be readily distinguished. However, in the reconstructed image with phase information, a tumor region may be more clearly distinguished from surrounding fibroglandular tissues, and a necrotic region may also be distinguished. Grating imaging advantageously supplements the limitations of grating-free two-dimensional absorption imaging. A combination of the two enables a more comprehensive analysis of the specimen.

As shown in FIG. 2, according to an embodiment of the present disclosure, a radiation imaging apparatus includes: a radiation source S configured to emit a radiation beam; a detector DT configured to receive the radiation beam from the radiation source S, where the radiation source S and the detector DT define an inspection channel; and one or more gratings arranged in the inspection channel and between the radiation source S and the detector DT. The one or more gratings are configured such that a portion of the radiation beam passes through the one or more gratings before being received by a first portion DT-1 of the detector, and another portion of the radiation beam is received by a second portion DT-2 of the detector without passing through the one or more gratings. In other words, as shown in FIG. 2, a grating is provided upstream of the first portion DT-1 of the detector, such that the radiation beam from the radiation source S first passes through the grating and is then detected by the detector, whereas no grating is provided upstream of the second portion DT-2 of the detector, such that the radiation beam received by the second portion DT-2 of the detector does not pass through any grating. The radiation beam ultimately detected by the detector may have passed through the object under inspection W. The one or more gratings may include one grating, two gratings, or three gratings as shown in FIG. 2. However, it should be understood that three gratings need not necessarily be provided upstream of a portion of the detector as shown in FIG. 2. FIG. 2 merely shows an implementation of the present disclosure.

In an embodiment of the present disclosure, as shown in FIG. 2, a portion (e.g., a left portion) of the radiation beam emitted by the radiation source S passes through grating G0 and/or G1 and/or G2, while another portion (e.g., a right portion) does not pass through any grating, and both portions are ultimately detected by the detector. According to such embodiments, an advantage is that the radiation beam emitted by the same radiation source S is used to inspect the same portion of the object under inspection W both with and without passing through a grating. This ensures that the radiation beams irradiating the object under inspection W are substantially identical, thereby avoiding differences or variations in the radiation beams caused by different radiation sources, which could otherwise lead to systematic errors or even misjudgments in the inspection. In such embodiments, since the same radiation source S is used to irradiate the object under inspection W, with one portion of the radiation beam passing through the grating and another portion not passing through the grating, an absorption image, a two-dimensional phase image, and a two-dimensional small-angle scattering image of at least a portion (e.g., a region of interest) of the object under inspection W may be obtained during a single pass of the object under inspection W. Accordingly, by comparing these acquired images, the properties and structures of, for example, biological tissues may be accurately determined.

In an embodiment, the detector may include two portions. For example, as shown in FIG. 2, the first portion DT-1 of the detector (a left portion in FIG. 2) includes a high-energy detector, and the second portion DT-2 of the detector (a right portion in FIG. 2) includes a low-energy detector. In such embodiments, since the radiation beam that passes through the grating is detected by the high-energy detector, while the radiation beam that does not pass through the grating is detected by the low-energy detector, the resulting two-dimensional absorption image may more clearly reflect the properties of at least a portion of the object under inspection W. According to such embodiments, the inventors have recognized that the grating absorbs a portion of the low-energy radiation energy to a certain extent. Therefore, a portion of the low-energy radiation beam does not reach the high-energy detector after passing through the grating, thereby reducing the influence of low-energy radiation beam on the high-energy detector. Consequently, the constructed high-energy two-dimensional absorption image may more clearly reflect the internal structure of the object under inspection W, which is particularly significant for biological specimens. Meanwhile, since the second portion DT-2 of the detector is configured as a low-energy detector, a low-energy two-dimensional absorption image of the object under inspection W constructed by the low-energy detector may also be provided. The high-energy absorption image and the low-energy absorption image may be combined to construct an information-enhanced comprehensive image, thereby providing more comprehensive two-dimensional absorption image information.

For an optical path portion including the grating (i.e., including the first portion DT-1 of the detector), when the X-ray beam passes through the grating, the absorption effect of the grating may harden the radiation energy spectrum, thereby increasing the average energy of the radiation. This allows for simultaneous acquisition of the absorption image, the phase image, and the small-angle scattering image, where the absorption image corresponds to the high-energy image in dual-energy imaging. For an optical path portion without any grating (i.e., including the first portion DT-2 of the detector), the X-ray beam passes through the object and is directly absorbed by the second portion DT-2 of the detector. Compared with the optical path portion including the first portion DT-1 of the detector, the average energy of the radiation is lower, and an absorption image may be obtained, where the absorption image corresponds to the low-energy image in dual-energy imaging.

On the one hand, the imaging system enables X-ray dual-energy imaging. Specifically, the high-energy absorption image from the first portion DT-1 of the detector and the low-energy absorption image from the second portion DT-2 of the detector may be combined, and layered imaging of multiple materials may be achieved through material decomposition, which facilitates material identification of the object under inspection. On the other hand, the imaging system enables multi-characteristic imaging (through DT-1). In addition to the ordinary absorption image, a phase image having higher contrast than the absorption image and a small-angle scattering image reflecting the microstructural characteristics of the object under inspection may also be obtained. Based on the foregoing imaging results, comprehensive X-ray imaging may be achieved, for example, through appropriate software and image processing techniques.

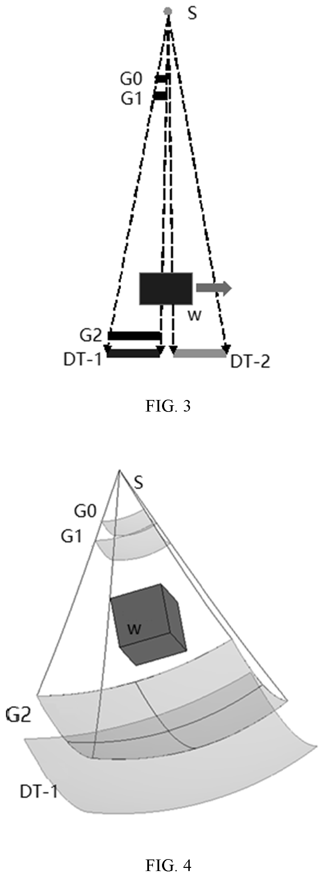

In an embodiment, the first portion DT-1 and the second portion DT-2 of the detector may be integrated, for example, as shown in FIG. 2. In another embodiment, the first portion DT-1 and the second portion DT-2 of the detector may be separated from each other, for example, as shown in FIG. 3.

In an embodiment of the present disclosure, the first portion DT-1 and the second portion DT-2 of the detector DT are the same type of detector, that is, both portions employ high-energy detectors and/or low-energy detectors. In such embodiments, the first portion DT-1 and the second portion DT-2 of the detector may be integrated or may be separated from each other.

According to embodiments of the present disclosure, the one or more gratings include a periodic structure used to diffract the radiation beam to generate an interference fringe. The grating may be a grating including a plurality of slits, that is, the periodic structure may include, for example, slits. Alternatively, the periodic structure may include other structures instead of slits, such as materials capable of generating fringe patterns. The periodic structure may be any periodic structure capable of generating an interference pattern or a moiré pattern after the radiation beam passes through the periodic structure. The grating may be a channel-type grating.

Taking the periodic structure including slits as an example, a periodic direction of the periodic structure may be an arrangement direction of the plurality of slits, while an extension direction of an individual substructure (e.g., a slit) of the periodic structure is perpendicular to the arrangement direction of the plurality of slits. According to the present disclosure, when the extension direction of an individual slit forms a very small angle (for example, relative to being parallel to the inspection channel), such as 10⁻⁴ degree, with the inspection channel (that is, the extension direction of the inspection channel, i.e., the direction indicated by the movement arrow of the object under inspection W in FIG. 2, namely the Z direction), the interference fringes or moiré fringes generated by the periodic structure formed by the arrangement of the plurality of slits may change significantly. Therefore, in the present disclosure, the requirements of the present disclosure may be satisfied as long as the extension direction of the individual slit is arranged at a very small angle relative to the inspection channel. In an embodiment, as shown in FIG. 2, the irradiation direction of the radiation beam is the Y direction, the extension direction of the inspection channel is the Z direction, and the movement direction of the object under inspection W is the Z direction. For example, the extension direction of an individual slit may be transverse to the extension direction of the inspection channel (i.e., perpendicular to the inspection channel). In such a case, the arrangement direction of the slits (i.e., the periodic direction of the periodic structure) is parallel to the extension direction of the inspection channel, and in FIG. 2, the arrangement direction of the slits extends horizontally from left to right (the Z direction). In other embodiments, the extension direction of an individual slit may form a non-zero angle with the extension direction of the inspection channel.

In an embodiment, the radiation imaging apparatus includes a grating G0, which is arranged adjacent to and parallel to the detector and is spaced apart from the detector by a first distance. The radiation beam irradiates the object under inspection W after passing through the grating G0. In other words, the grating G0 is located upstream of the object under inspection W.

In an embodiment, the radiation imaging apparatus further includes a grating G1, which is different from the grating G0. That is, two gratings, namely grating G0 and grating G1, are provided in such embodiments. The grating G1 is arranged adjacent to and parallel to the detector and is spaced apart from the detector by a second distance, and the second distance is different from the first distance. The radiation beam irradiates the object under inspection W after passing through the grating G1, that is, the grating G1 is also located upstream of the object under inspection W. In such embodiments, the two gratings G0 and G1 are provided upstream of the object under inspection W.

In an embodiment, the radiation imaging apparatus includes a grating G0, a grating G1, and a grating G2. In such embodiments, the grating G2 is located downstream of the object under inspection W, that is, the radiation beam first passes through the object under inspection W and then passes through the grating G2. In such embodiments, the object under inspection W passes between the grating G1 and the grating G2. In another embodiment, the object under inspection W passes between the grating G0 and the grating G1, and the grating G1 and the grating G2 are both located downstream of the object under inspection W. A fourth distance between the grating G2 and the detector is different from a second or third distance between the grating G1 and the detector.

In an embodiment of the present disclosure, the grating may have a grating surface. In an embodiment, the grating may have an arcuate surface. FIG. 4 shows an embodiment in which the gratings G0, G1, and G2 each have an arcuate surface. For simplicity, FIG. 4 shows only the portion of the present disclosure including the gratings. In such embodiments, the detector may also have an arcuate surface as shown in FIG. 4.

According to an aspect of the present disclosure, a CT imaging apparatus is provided. In such embodiments, the CT imaging apparatus includes a rotating ring configured to be rotatable. The imaging apparatus further includes the radiation imaging apparatus as described above, which is arranged on the rotating ring and configured to rotate with the rotating ring. Through rotation of the rotating ring, the radiation source may irradiate the object under inspection W from different angles, thereby enabling acquisition of images of different tomographic sections. The acquired images may be processed by a computer to construct a three-dimensional image of the object under inspection W as well as a three-dimensional grating image. The CT imaging apparatus may further include other components, such as a processor or a computing unit, which will not be described in detail herein.

According to an aspect of the present disclosure, a radiation imaging method is provided, including: imaging the object under inspection W using the radiation imaging apparatus as described above, where the object under inspection W moves along the inspection channel to sequentially pass through the first portion DT-1 and the second portion DT-2 of the detector, and at least one of an absorption image, a phase image, or a small-angle scattering image related to at least a partial region of the object under inspection W is generated based on signals detected by the first portion DT-1 and the second portion DT-2 of the detector.

According to an aspect of the present disclosure, a CT imaging method is provided, including: imaging the object under inspection W using the CT imaging apparatus as described above, where the object under inspection W moves along the inspection channel to sequentially pass through the first portion DT-1 and the second portion DT-2 of the detector, and at least one of a three-dimensional absorption image, a three-dimensional phase image, or a three-dimensional small-angle scattering image related to at least a partial region of the object under inspection W is generated based on signals detected by the first portion DT-1 and the second portion DT-2 of the detector.

It should be understood that the various forms of processes shown above may be used, with steps reordered, added, or deleted. For example, the steps described herein may be performed in parallel, sequentially, or in a different order, as long as the desired results of the technical solutions provided by the present disclosure may be achieved, and no limitation is imposed herein.

In the technical solutions of the present disclosure, the collection, storage, use, processing, transmission, provision, disclosure, etc., of user personal information involved all comply with relevant laws and regulations and do not violate public order and good morals.

The foregoing specific embodiments are not intended to limit the scope of protection of the present disclosure. Those skilled in the art will appreciate that various modifications, combinations, sub-combinations, and substitutions may be made to the disclosed embodiments or examples according to design requirements and other factors. Any modification, equivalent substitution, and improvement within the spirit and principle of the present disclosure shall fall within the scope of protection of the present disclosure.

## Claims

1. A radiation imaging apparatus, comprising:
a radiation source configured to emit a radiation beam;
a detector configured to receive the radiation beam from the radiation source, wherein the radiation source and the detector define an inspection channel; and
one or more gratings arranged in the inspection channel and between the radiation source and the detector;
wherein the one or more gratings are configured such that a portion of the radiation beam passes through the one or more gratings before being received by a first portion of the detector, and another portion of the radiation beam is received by a second portion of the detector without passing through the one or more gratings.

2. The radiation imaging apparatus of claim 1, wherein the first portion of the detector comprises a high-energy detector, and the second portion of the detector comprises a low-energy detector.

3. The radiation imaging apparatus of claim 2, wherein the radiation imaging apparatus is configured to construct at least a low-energy absorption image based on a radiation beam signal acquired by the low-energy detector, construct a high-energy absorption image based on a radiation beam signal acquired by the high-energy detector, and combine the high-energy absorption image and the low-energy absorption image to construct an information-enhanced comprehensive image.

4. The radiation imaging apparatus of claim 3, wherein the one or more gratings comprise a periodic structure, and the periodic structure is configured to diffract the radiation beam to generate an interference fringe.

5. The radiation imaging apparatus of claim 1, wherein the first portion of the detector and the second portion of the detector are integrated, or the first portion of the detector and the second portion of the detector are separated from each other.

6. The radiation imaging apparatus of claim 1, wherein the one or more gratings comprise:
a first grating arranged adjacent to and parallel to the detector and spaced apart from the detector by a first distance, wherein the radiation beam irradiates an object under inspection after passing through the first grating; and/or
a second grating arranged adjacent to and parallel to the detector and spaced apart from the detector by a second distance different from the first distance, wherein the radiation beam irradiates the object under inspection after passing through the second grating; or a second grating arranged adjacent to and parallel to the detector and spaced apart from the detector by a third distance, wherein the radiation beam passes through the second grating after passing through the object under inspection; and/or
a third grating arranged adjacent to and parallel to the detector and spaced apart from the detector by a fourth distance different from the third distance, wherein the radiation beam passes through the third grating after passing through the object under inspection.

7. The radiation imaging apparatus of claim 7, comprising a first grating, a second grating, and a third grating, wherein the first grating, the second grating, and the third grating are parallel to each other.

8. The radiation imaging apparatus of claim 1, wherein a periodic structure of the one or more gratings is a channel-type structure or a slit-type structure.

9. The radiation imaging apparatus of claim 1, wherein the radiation imaging apparatus is configured to generate, based on signals detected by the detector, at least one of an absorption image, a phase image, or a small-angle scattering image related to at least a partial region of an object under inspection in the inspection channel.

10. The radiation imaging apparatus of claim 1, wherein the one or more gratings comprise a planar grating or an arcuate surface grating.

11. A CT imaging apparatus, comprising:
a rotating ring configured to be rotatable; and
the radiation imaging apparatus of any one of claims 1 to 10, wherein the radiation imaging apparatus is arranged on the rotating ring and configured to rotate with the rotating ring.

12. A radiation imaging method, comprising:
imaging an object under inspection using the radiation imaging apparatus of any one of claims 1 to 10, wherein the object under inspection moves along the inspection channel to sequentially pass through the first portion of the detector and the second portion of the detector, and at least one of an absorption image, a phase image, or a small-angle scattering image related to at least a partial region of the object under inspection is generated based on signals detected by the first portion of the detector and the second portion of the detector.

13. A CT imaging method, comprising:
imaging an object under inspection using the CT imaging apparatus of claim 11,
wherein the object under inspection moves along the inspection channel to sequentially pass through the first portion of the detector and the second portion of the detector, and at least one of a three-dimensional absorption image, a three-dimensional phase image, or a three-dimensional small-angle scattering image related to at least a partial region of the object under inspection is generated based on signals detected by the first portion of the detector and the second portion of the detector.
